# EUROPÄISCHE PATENTSCHRIFT

(11) **EP 2 173 399 B1**
(45) Veröffentlichungstag und Bekanntmachung des Hinweises auf die Patenterteilung: **02.01.2013**
(21) Anmeldenummer: 08773810.0
(22) Anmeldetag: 02.07.2008
(51) Int. Cl.: A61L 31/12, A61L 31/14, A61L 31/16, A61L 27/18, A61L 27/48, A61L 33/06

(54) **GEFÄSSPROTHESE MIT REDUZIERTER THROMBOGENITÄT**
VASCULAR PROSTHESIS WITH REDUCED THROMBOGENICITY
PROTHÈSE VASCULAIRE À THROMBOGÉNICITÉ RÉDUITE

(30) Priorität: 09.07.2007 DE 102007033787
(43) Veröffentlichungstag der Anmeldung: 14.04.2010
(73) Patentinhaber: Aesculap AG, 78532 Tuttlingen/Donau (DE)
(72) Erfinder: LANGANKE, Dennis, 78532 Tuttlingen (DE); GOLDMANN, Helmut, 78532 Tuttlingen (DE)
(74) Vertreter: Patentanwälte Ruff, Wilhelm, Beier, Dauster & Partner
(86) Internationale Anmeldenummer: PCT/EP2008/005396
(87) Internationale Veröffentlichungsnummer: WO 2009/007046

(56) Entgegenhaltungen:
- DE-A1- 3 131 071
- DE-A1- 19 955 341
- US-A1- 2001 044 655
- US-A1- 2002 028 201

## Beschreibung

Die vorliegende Erfindung betrifft eine Gefäßprothese mit verbesserten antithrombogenen Eigenschaften sowie ein diesbezügliches Herstellungsverfahren.

Beim Verschluss eines peripheren arteriellen Gefäßes, beispielsweise im Ober- oder Unterschenkel, wird häufig zur Umgehung der verschlossenen Gefäßstrecke ein Bypass angelegt. Hierfür eignet sich aufgrund der langfristig guten Offenheitsrate vor allem autologes Material, insbesondere die körpereigene Vene. Sofern Venenmaterial nicht zur Verfügung steht, muss auf ein künstliches alloplastisches Bypassmaterial zurückgegriffen werden. Diese sogenannten Gefäßprothesen haben sich ebenfalls in der klinischen Praxis bewährt. Hergestellt werden sie bevorzugt aus Polyester, insbesondere Polyethylenterephthalat (PET), oder aus expandiertem Polytetrafluorethylen (ePTFE). Aus Polyester werden in der Regel textile Gefäßprothesen hergestellt, wohingegen ePTFE das Ausgangsmaterial zur Herstellung von sogenannten non-woven Gefäßprothesen darstellt.

Im Gegensatz zur Vene kann es bei den Gefäßprothesen häufiger und zeitlich früher zu einem Verschluss des Bypasses kommen. Eine Ursache für dieses Prothesenversagen stellt das sogenannte Compliance-Miss-Match dar. Hierunter wird der Unterschied im Elastizitätsmodul zwischen dem Empfängergefäß und der Gefäßprothese verstanden. Hierdurch kann es im Bereich der Verbindungsstelle zwischen natürlichem Gefäß und Prothese zu einer Proliferation von glatten Muskelzellen kommen, was schließlich zum Verschluss des Gefäßes führen kann. Eine weitere Ursache für den Verschluss der Prothese stellt die Thrombogenität des Bypassmaterials dar. Hier kann zwischen einer Strukturthrombogenität und einer Materialthrombogenität unterschieden werden. Textile Gefäßprothesen sind aufgrund ihrer textilen Struktur und der hohen Strukturthrombogenität nur noch bedingt für den Ersatz von Gefäßen mit einem Durchmesser < 6 mm geeignet. Bessere Ergebnisse, d.h. eine bessere Offenheitsrate, werden hier insbesondere mit Gefäßprothesen aus ePTFE erzielt. Im Vergleich zur autologen Vene ist jedoch die Offenheitsrate der Gefäßprothesen aus ePTFE aufgrund der Materialthrombogenität sowie des Compliance-Miss-Match deutlich geringer als die der autologen Vene.

Der Thrombus innerhalb einer Gefäßprothese entsteht insbesondere durch Wechselwirkungen des Blutes mit der Innenwand der Prothese. Hierbei kommt es zur Aktivierung von plasmatischen Gerinnungsfaktoren und zur Adhäsion von Blutplättchen, beispielsweise Thrombozyten, an der Prothesenwand, was den Beginn eines thrombotischen Verschlusses darstellt.

Daher wird ständig an einer Verbesserung der Prothesenleistung im Hinblick auf die Verminderung des Compliance-Miss-Match und der Vermeidung einer Thrombenbildung gearbeitet. So kommen beispielsweise auch Gefäßprothesen aus Polyurethan zum Einsatz, die gegenüber ePTFE-Gefäßprothesen gewisse Vorteile hinsichtlich der Elastizität und der Compliance aufweisen. Beispielsweise geht eine derartige Gefäßprothese aus der DE 28 06 030 C2 hervor.

Ein weiterer Ansatz zur Verbesserung der antithrombogenen Eigenschaften von Gefäßprothesen besteht unter anderem darin, deren Innenwandoberflächen möglichst glatt und insbesondere geschlossen auszubilden, so dass Blutbestandteile daran schlechter adhärieren können.

In der Oberflächentechnologie werden verschiedene Beschichtungen ausprobiert, um die Anhaftung von Blutbestandteilen auf Oberflächen zu reduzieren. So werden in der DE 199 55 341 A1 geeignete Polysiloxanbeschichtungen beschrieben, um die Anlagerung von Thrombozyten auf Kunststoffoberflächen zu vermindern.

In der US 2002/0028201 A1 wird ein interaktives System, beispielsweise in Form einer Gefäßprothese, auf Basis einer aktiven Kunststoffoberfläche mit einem Substanz-Linker-Konjugat beschrieben, welches insbesondere der gerichteten Immobilisierung und Präsentation physiologischer Substanzen in flüssigen Medien dient.

Gefäßprothesen mit einer bioaktiven Beschichtung werden ferner in der US 2001/0044655 A1 beschrieben, wobei die Beschichtung ein Konjugat aus einem Linker und einem bioaktiven Molekül wie beispielsweise Heparin umfasst.

Die vorliegende Erfindung stellt sich somit die Aufgabe, eine Gefäßprothese, insbesondere für den arteriellen Gefäßersatz im kleinlumigen Bereich, mit verbesserten antithrombogenen Eigenschaften bereitzustellen.

Diese Aufgabe wird gelöst durch eine Gefäßprothese mit einer porösen Wand aus synthetischem Polymer, wobei in die Innenwand der Prothese Mikropartikel eingelagert sind, auf deren Oberflächen Blutgerinnungsinhibitoren immobilisiert sind.

Die Blutgerinnungsinhibitoren sind vorzugsweise über sogenannte Linker (Spacermoleküle) auf den Oberflächen der Mikropartikel immobilisiert. In der Regel sind die Linker nicht kovalent, vorzugsweise adsorptiv, an die Mikropartikel gebunden. Die Blutgerinnungsinhibitoren sind bevorzugt kovalent mit den Linkern verknüpft. Die kovalente Verknüpfung basiert gewöhnlich auf einer chemischen Kondensationsreaktion zwischen funktionellen Gruppen, beispielsweise Hydroxy- und/oder Aminogruppen, der Linker und geeigneten reaktiven Gruppen der Inhibitoren. Durch die Verknüpfung mit den Linkern befinden sich die Blutgerinnungsinhibitoren in einem gewissen Abstand zu den Mikropartikeln. Dadurch können Aktivitätsbeeinträchtigungen der Inhibitoren weitgehend vermieden werden. Die Immobilisierung der in diesem Abschnitt beschriebenen Linker-Inhibitor-Konjugate auf den Mikropartikeloberflächen beruht vorzugsweise auf adsorptiven, insbesondere elektrostatischen Wechselwirkungen, zwischen den Linkern und den Mikropartikeloberflächen.

In einer bevorzugten Ausführungsform handelt es sich bei den Linkern um Polymermoleküle, die zweckmäßigerweise linear gebaut sind. Bevorzugt handelt es sich bei den Linkern um Oligo- oder Polyalkylenglykole, insbesondere Polyethylenglykol (PEG).

Bei den Blutgerinnungsinhibitoren handelt es sich vorzugsweise um Serinproteaseinhibitoren, insbesondere um Thrombininhibitoren. Thrombin ist das zentrale Enzym der plasmatischen Blutgerinnung, welches Fibrinogen zu monomerem Fibrin spaltet. Dieses polymerisiert anschließend und vernetzt an der Gefäßinnenwand adhärierte Blutbestandteile zu einem Thrombus.

In einer besonders bevorzugten Ausführungsform sind die Blutgerinnungsinhibitoren synthetische Inhibitoren, insbesondere Inhibitoren mit einer Oligo- oder Dipeptidstruktur. Vorzugsweise handelt es sich um Oligo- oder Dipeptide, insbesondere Dipeptide. Diesbezüglich wird auf die DE 101 02 878 A1 Bezug genommen, deren Offenbarungsgehalt vollumfänglich durch Bezugnahme zum Inhalt der vorliegenden Beschreibung gemacht wird.

In einer weiteren Ausführungsform handelt es sich bei den Blutgerinnungsinhibitoren um endogene, d.h. um im menschlichen oder tierischen Körper vorkommende, Inhibitoren. Erfindungsgemäß kommen insbesondere Antithrombine, insbesondere Antithrombin III, in Betracht. Antithrombine sind die natürlichen Gegenspieler von Thrombin. Des weiteren kann es sich bei den Inhibitoren um Hirudin handeln. Hirudin ist ein Polypeptid, welches an die Fibrinogenbindestelle von Thrombin bindet, wodurch dessen Wirkung inhibiert wird. Weiterhin kann es sich bei den Inhibitoren um bestimmte Polysaccharide, insbesondere Heparin, handeln. Beispielsweise ist das Polysaccharid ein Heparin mit einem Molekulargewicht von ca. 17.000 Dalton.

Erfindungsgemäß ist es weiterhin möglich, dass die Blutgerinnungsinhibitoren rekombinanter Natur sind. Beispielsweise kann das im Rahmen der vorliegenden Erfindung verwendbare Hirudin rekombinant hergestellt sein.

In einer weiteren vorteilhaften Ausführungsform können auf den Oberflächen der Mikropartikel verschiedene Blutgerinnungsinhibitoren immobilisiert sein. Beispielsweise können auf den Mikropartikeln sowohl Antithrombin III als auch Heparin immobilisiert sein. Dies ist besonders vorteilhaft, da Antithrombin III durch die Anwesenheit von Heparin in seiner Aktivität verstärkt und insbesondere beschleunigt wird.

Gemäß einer weiteren Ausführungsform sind die immobilisierfähigen Stellen der Mikropartikel vollständig mit den Blutgerinnungsinhibitoren abgesättigt.

Die Mikropartikel können weiterhin aus einem nicht resorbierbaren Material gebildet sein. Bevorzugt handelt es sich bei den Mikropartikeln der erfindungsgemäßen Gefäßprothese um polymere Mikropartikel. Dabei können die Mikropartikel aus einem anderen Polymer als die Prothesenwand gebildet sein. Ein bevorzugtes Polymer, welches im Rahmen der vorliegenden Erfindung eingesetzt werden kann, ist ein Polyalkylacrylat, vorzugsweise Polyalkylmethacrylat (PAMA), mit einem Alkylrest, der vorzugsweise 1 bis 6 Kohlenstoffatome umfasst. Die Mikropartikel können beispielsweise aus Polymethylmethacrylat (PMMA), Polyethylmethacrylat (PEMA) oder Polypropylmethacrylat gebildet sein. Als weitere mögliche Polymere kommen Polyvinylacetat, Polycyclohexylmethacrylat oder Polyphenylmethacrylat in Betracht. Polymethylmethacrylat-Mikropartikel sind besonders bevorzugt.

Die Mikropartikel sind ausschließlich an der Innenseite, vorzugsweise in einer Innenschicht, der Prothesenwand eingelagert. Die Prothesenwandinnenseite ist bevorzugt bis zu einer Schichtdicke von 0,01 bis 0,2 mm mit den Mikropartikeln beladen. Die Mikropartikel können mit der Prothesenwand verbunden, insbesondere verklebt sein.

In einer weitergehenden Ausführungsform ist die Innenseite der Prothesenwand aus einer Schicht der Mikropartikel gebildet. Die Partikeldichte in der Prothesenwand beträgt vorzugsweise 0,2 bis 20 %, bezogen auf die Fläche der Prothesenwand. Der Anteil an Mikropartikeln in der erfindungsgemäßen Gefäßprothese beträgt 1,5 bis 30 Gew.-%, bezogen auf das Gesamtgewicht der Prothese. Die Mikropartikel können weiterhin eine Größe bzw. einen Durchmesser von 1 bis 200 µm, vorzugsweise von 5 bis 60 µm, aufweisen.

In einer zweckmäßigen Ausführungsform ist die Prothese eine textile Gefäßprothese. Bevorzugt handelt es sich bei der erfindungsgemäßen Gefäßprothese um eine non woven-Prothese, insbesondere eine Vlies-Prothese. Die Prothesenwand kann insbesondere von einem Sprühvlies gebildet sein. Mit anderen Worten kann die Gefäßprothese eine Sprühvlies-Prothese sein.

In einer weiteren Ausführungsform ist die Prothesenwand aus einem synthetischen und vorzugsweise nicht resorbierbaren Polymer gebildet. Vorzugsweise ist die Prothesenwand im wesentlichen aus Polyurethan, insbesondere unvernetztem Polyurethan, gebildet. Bei dem Polyurethan kann es sich um ein aliphatisches Polyurethan handeln, welches bevorzugt aus makromolekularen und/oder niedermolekularen aliphatischen Diolen und aliphatischen Diisocyanaten gebildet ist. Bevorzugt kommen als makromolekulare Diole Polycarbonate, insbesondere 1,6-Hexandiolpolycarbonat, in Frage. Bei den niedermolekularen Diolen handelt es sich insbesondere um 2,2,4-Trimethylhexandiol, 2,4,4-Trimethylhexandiol und/oder 1,4-Butandiol. Als aliphatische Diisocyanate kommen vorzugsweise cycloaliphatische Diisocyanate, insbesondere 4,4'-Dicyclohexylmethandiisocyanat oder 1,4-Cyclohexyldiisocyanat, in Frage. Erfindungsgemäß ist es weiterhin möglich, dass das Polyurethan aus verschiedenen Diolen und/oder Diisocyanaten hergestellt ist. Bezüglich weiterer Einzelheiten und Eigenschaften von Polyurethanen wird auf die DE 36 43 465 A1, DE 33 18 730 A1, DE 41 07 284 A1 sowie auf den Polymerbericht "Biocompatible poly-urethanes for medical techniques" des Forschungsinstituts der Enka AG in Obernberg verwiesen, deren Offenbarungsgehalt jeweils durch Bezugnahme vollumfänglich zum Inhalt der vorliegenden Erfindung gemacht wird.

Das Polyurethan kann ein Molekulargewicht von 5.000 bis 50.000 Dalton, insbesondere von 20.000 bis 40.000 Dalton, besitzen.

In einer weiteren Ausführungsform ist die Innenseite der Prothesenwand aus einem anderen Polymer gebildet als die übrige Wand der erfindungsgemäßen Gefäßprothese. So kann beispielsweise die Innenseite der Prothesenwand aus Polyurethan und die Außenseite der Prothesenwand aus einem Polyester, insbesondere aus Polyethylenterephthalat (PET), gebildet sein. Bevorzugt liegt der Polyester bei der zuletzt genannten Ausführungsform in Form eines gewirkten Netzes vor.

Die Wandstärke der Gefäßprothese liegt bevorzugt zwischen 0,1 und 1,0 mm, insbesondere zwischen 0,2 und 0,6 mm. Grundsätzlich kann die Gefäßprothese einen Innendurchmesser zwischen 2 und 40 mm aufweisen. Bevorzugt weist die Prothese einen Innendurchmesser zwischen 2 und 12 mm, insbesondere 2 und 10 mm, auf. Gefäßprothesen mit derartigen Innendurchmesser werden bevorzugt für den kleinlumigen Gefäßersatz verwendet. Die radiale Reißkraft der Prothese beträgt vorzugsweise 2 bis 4 N/mm. Die Reißdehnung kann insbesondere 400 bis 600% betragen.

Die erfindungsgemäße Gefäßprothese besitzt vorteilhafterweise eine Porosität von 1 bis 200 ml Luft/cm²/min, insbesondere von 15 bis 50 ml Luft/cm²/min, bei einer Druckdifferenz von 1,2 kPas.

Die Gefäßprothese kann weiterhin in sterilisierter Form vorliegen. Zur Sterilisierung der zweckmäßigerweise in verpacktem Zustand vorliegenden Gefäßprothese eignet sich insbesondere Elektronenstrahlung. Die Expositionsdauer der Prothese liegt vorzugsweise im Sekundenbereich. Gewöhnlich ist eine Bestrahlung ausreichend, um eine sterilisierte Prothese zu erhalten. Grundsätzlich ist aber auch eine mehrmalige Bestrahlung denkbar. Die Strahlungsdosis beträgt bevorzugt ca. 25 kGray.

Die vorliegende Erfindung betrifft weiterhin ein Herstellungsverfahren für die erfindungsgemäße Gefäßprothese, wobei die Mikropartikel bei der Herstellung der Prothesenwand in diese integriert werden und die Mikropartikel nach Fertigstellung der Prothesenwand mit den Blutgerinnungsinhibitoren beladen werden.

Zunächst wird eine die Mikropartikel enthaltende Polymerdispersion auf einen insbesondere rotierbaren Formkern unter Ausbildung einer porösen Schicht, die die Innenseite der Prothesenwand bildet, aufgebracht, vorzugsweise aufgesprüht. Bevorzugt wird hierfür eine die Mikropartikel dispergiert enthaltende Polymerlösung verwendet. Danach wird die restliche Prothesenwand als keine Mikropartikel enthaltendes Sprühvlies ausgebildet.

Als geeignete Dispersionsmittel kommen insbesondere organische Lösungsmittel, vorzugsweise halogenierte Lösungsmittel, in Betracht. Bevorzugte Lösungsmittel, die im Rahmen der vorliegenden Erfindung verwendet werden können, sind chlorierte Lösungsmittel, beispielsweise Chloroform und/oder Dichlormethan.

Als Formkern wird zweckmäßigerweise ein rotationssymmetrischer, insbesondere zylindrischer, Kern verwendet. Der Formkern kann weiterhin mindestens eine Krümmung aufweisen. So kann der Formkern beispielsweise U-förmig ausgebildet sein. Als Formkern wird insbesondere ein Stab oder eine Walze eingesetzt. Der Formkern selbst kann aus einem Metall oder Kunststoff, beispielsweise aus PTFE oder Polyethylen, bestehen. Gewöhnlich wird ein Formkern aus Stahl eingesetzt. Der Formkern kann einen Durchmesser zwischen 2 und 40 mm aufweisen. Im Hinblick auf die Herstellung von kleinlumigen Gefäßprothesen weist der verwendete Formkern typischerweise einen Durchmesser zwischen 2 und 20 mm, insbesondere zwischen 4 und 12 mm, auf. Der Formkern kann weiterhin vor der Herstellung der Gefäßprothese mit einer Folie oder einem Schlauch, beispielsweise einem Latexschlauch, überzogen werden. Auf diese Weise lässt sich die hergestellte Gefäßprothese leichter von dem Formkern abziehen.

Das Aufbringen der Mikropartikeldispersion bzw. der die Mikropartikel enthaltenden Polymerdispersion auf den Formkern wird zweckmäßigerweise mit Hilfe einer Sprüh- oder Zerstäubervorrichtung vorgenommen. Besonders bevorzugt erfolgt das Aufbringen mittels mindestens einer Sprühpistole oder -düse. Erfindungsgemäß kann es insbesondere vorgesehen sein, dass zur Herstellung der Innenseite der Prothesenwand 10 bis 30 Auftragszyklen, insbesondere ca. 20 Auftragszyklen, durchgeführt werden. Die Dispersion kann beispielsweise mittels Druck oder Pressluft, insbesondere von ca. 0,5 bar, in Richtung des Formkerns versprüht werden. Bezüglich weiterer Einzelheiten sowie Merkmale zur Sprühtechnik wird vollumfänglich auf die DE 28 57 925 C2 sowie auf die DE 31 31 071 A1 verwiesen, deren Offenbarungsgehalt jeweils durch Bezugnahme zum Inhalt der vorliegenden Beschreibung gemacht wird.

Durch Einstellen des Winkels zwischen Formkern und Auftragsvorrichtung kann die Faserausrichtung in der Prothesenwand eingestellt werden, wobei insbesondere ein Winkel zwischen 45 ° und 60 ° gewählt wird.

Das Aufbringen der Polymerdispersion wird vorzugsweise aus einer Entfernung zum Formkern vorgenommen, die eine Faserbildung des Polymermaterials aus der Dispersion beim Durchlaufen der Aufbringungsstrecke (Auftragstrecke) zur Erzeugung einer Vliesstruktur ermöglicht. Die Polymerdispersion kann insbesondere aus mehreren und vorzugsweise verschiedenen Entfernungen zum Formkern aufgetragen werden, wobei die Entfernungen im Verhältnis zueinander bevorzugt ansteigend sind.

Weiterhin kann die Aufbringungsstrecke zur Herstellung der Prothesenwand kontinuierlich vergrößert werden, wodurch eine Prothesenwand erzeugt wird, welche über ihren Querschnitt eine graduelle Veränderung der Porenanzahl und/oder -durchmesser aufweist, wobei die Porenanzahl und/oder -durchmesser vorzugsweise von der Innenseite zur Außenseite der hergestellten Prothesenwand kontinuierlich zunehmen.

Weiterhin kann das Aufbringen der Polymerdispersion, insbesondere zusammen mit den Mikropartikeln, aus einer Entfernung zum Formkern vorgenommen werden, welche eine Faserbildung des Polymermaterials beim Durchlaufen der Aufbringungsstrecke verhindert. Auf diese Weise kann eine glatte und insbesondere dichte (geschlossene) Prothesenwand, vorzugsweise in Form einer Folie, erzeugt werden. Diese Ausführungsform kommt bevorzugt zur Herstellung einer Innenseite der erfindungsgemäßen Gefäßprothese zum Einsatz, die eine hohe Beladungsdichte an Mikropartikeln an der Innenoberfläche aufweist.

Das Aufbringen der Mikropartikeldispersion bzw. Polymerdispersion erfolgt typischerweise aus einer Entfernung zum Formkern von 10 bis 75 cm, insbesondere 15 bis 50 cm. Zur Herstellung einer glatten und insbesondere dichten Prothesenwand kann die Polymerdispersion aus einer Entfernung von 1 bis 15 cm, insbesondere 4 bis 8 cm, aufgetragen werden.

Zur Beladung der Mikropartikel mit den Blutgerinnungsinhibitoren lässt man vorteilhafterweise eine die Inhibitoren enthaltende Dispersion, vorzugsweise eine die Inhibitoren enthaltende Lösung, durch die Prothesenwand fließen. Besonders bevorzugt enthält die Dispersion bzw. Lösung Linker-Inhibitor-Konjugate.

Weitere Einzelheiten und Merkmale der vorliegenden Erfindung ergeben sich aus der nachfolgenden Beschreibung einer bevorzugten Ausführungsform in Form eines Beispiels, welches die Erfindung in keiner Weise einschränken soll.

### Beispiel:

### Herstellung einer kleinlumigen Gefäßprothese mit einem an der Prothesenwandinnenseite immobilisierten Thrombininhibitor

In einem Ultraschallbad werden 4 g Polymethylmethacrylat-Mikropartikel und 200 ml einer Polyurethan-Lösung in Chloroform (10 Gew.-% Polyurethan) während 15 Minuten homogenisiert. Die so erhaltene Dispersion wird mit einem Druck von 0,6 bar mittels einer Sprühpistole (z.B. Krautzberger Typ A-11) auf eine rotierende Metallstange gesprüht. Zum Erhalt der mit Mikropartikeln versetzten Innenoberfläche werden ca. 20 Sprühzyklen bei einem Sprühabstand von ca. 40 cm versprüht. Danach wird die Sprühlösung gewechselt. In einen Rührtopf werden ca. 1.200 g Polyurethan und 9.000 ml Chloroform gegeben und mit ca. 260 U/min. während 72 Stunden gerührt. Die erhaltene Lösung wird durch Druckluft (0,5 bar) mit Hilfe der vorstehend erwähnten Sprühpistole versprüht. Die Sprühpistole steht in einem Winkel von ca. 45 ° zu einer mit 300 U/min. rotierenden Metallstange. Die Materialzuführung zu der Pistole erfolgt mit Hilfe einer Zahnradpumpe. Durch drei weitere Sprühphasen wird eine mikroporöse Struktur der Prothese erreicht, wobei jeweils 30 Sprühzyklen mit unterschiedlichen Abständen zwischen 5 und 24 cm gesprüht werden. Durch den geringfügigen Restgehalt an Chloroform an den Einzelfasern kommt es an den Kreuzungspunkten der auf die Metallstange aufgetragenen Einzelfasern zu Verklebungen. Es wird eine Faserstruktur ausgebildet, welche die Mikrostruktur der Prothese bildet. Nach Beendigung des Sprühvorganges wird die Metallstange mit der darauf gesprühten Gefäßprothese für ca. 10 Sekunden in reines Isopropanol getaucht, so dass die Prothese in ihrer Gesamtheit vollständig benetzt wird. Nach einer Abtropfzeit von 5 Minuten wird die Metallstange mit der darauf befindlichen Gefäßprothese rotierend getrocknet. Danach wird die Gefäßprothese von der Metallstange abgenommen und eine Lösung mit einem Konjugat aus PEG und einem synthetischen Thrombininhibitor durch die einseitig verschlossene Gefäßprothese über 150 Minuten lang im Kreislauf durch die Prothesenwand gepumpt. Das PEG-Thrombininhibitor-Konjugat kommt dabei zuerst mit der Innenschicht der Prothesenwand in Kontakt, welche die Mikropartikel enthält.

## Patentansprüche

1. Gefäßprothese mit einer porösen Wand aus synthetischem Polymer, **dadurch gekennzeichnet, dass** in die Wand Mikropartikel eingelagert sind, auf deren Oberflächen Blutgerinnungsinhibitoren immobilisiert sind, wobei die Mikropartikel ausschließlich an der Innenseite der Prothesenwand eingelagert sind und der Anteil an Mikropartikeln in der Prothese 1,5 bis 30 Gew.-% beträgt.

2. Gefäßprothese nach Anspruch 1, **dadurch gekennzeichnet, dass** die Blutgerinnungsinhibitoren über Linker auf den Mikropartikeloberflächen immobilisiert sind, wobei vorzugsweise es sich bei den Linkern um Polyalkylenglykole, insbesondere Polyethylenglykol (PEG), handelt.

3. Gefäßprothese nach Anspruch 1 oder 2, **dadurch gekennzeichnet, dass** es sich bei den Blutgerinnungsinhibitoren um endogene Inhibitoren, insbesondere Antithrombin III und/oder Hirudin, handelt.

4. Gefäßprothese nach einem der vorhergehenden Ansprüche, **dadurch gekennzeichnet, dass** die Mikropartikel aus Polyalkylacrylat, insbesondere Polymethylmethacrylat, gebildet sind.

5. Gefäßprothese nach einem der vorhergehenden Ansprüche, **dadurch gekennzeichnet, dass** die Mikropartikel ausschließlich in einer Innenschicht der Prothesenwand eingelagert sind, wobei vorzugsweise die Prothesenwandinnenseite bis zu einer Schichtdicke von 0,01 bis 0,2 mm mit den Mikropartikeln beladen ist.

6. Gefäßprothese nach einem der vorhergehenden Ansprüche, **dadurch gekennzeichnet, dass** die Innenseite der Prothesenwand aus einer Schicht aus Mikropartikeln gebildet ist.

7. Gefäßprothese nach einem der vorhergehenden Ansprüche, **dadurch gekennzeichnet, dass** die Mikropartikel eine Größe von 1 bis 200 µm, vorzugsweise 5 bis 60 µm, haben.

8. Gefäßprothese nach einem der vorhergehenden Ansprüche, **dadurch gekennzeichnet, dass** die Prothese eine textile Gefäßprothese, vorzugsweise eine non-woven-Prothese, ist.

9. Gefäßprothese nach einem der vorhergehenden Ansprüche, **dadurch gekennzeichnet, dass** die Prothesenwand von einem Sprühvlies gebildet wird.

10. Gefäßprothese nach einem der vorhergehenden Ansprüche, **dadurch gekennzeichnet, dass** die Prothesenwand im wesentlichen aus Polyurethan, insbesondere unvernetztem Polyurethan, gebildet ist.

11. Gefäßprothese nach einem der vorhergehenden Ansprüche, **dadurch gekennzeichnet, dass** die Innenseite der Prothesenwand aus einem anderen Polymer gebildet ist als die übrige Wand.

12. Verfahren zur Herstellung der Gefäßprothese nach einem der vorhergehenden Ansprüche, **dadurch gekennzeichnet, dass** die Mikropartikel bei der Herstellung der Prothesenwand in diese integriert werden und die Mikropartikel nach Fertigstellung der Prothesenwand mit den Blutgerinnungsinhibitoren beladen werden, wobei zunächst eine die Mikropartikel enthaltende Polymerdispersion auf einen insbesondere rotierbaren Formkern unter Ausbildung einer porösen Schicht, die die Innenseite der Prothesenwand bildet, aufgebracht, vorzugsweise aufgesprüht, wird und danach die restliche Prothesenwand als keine Mikropartikel enthaltendes Sprühvlies ausgebildet wird.

## Claims

1. Vascular prosthesis having a porous wall composed of synthetic polymer, **characterized in that** the wall is embedded with microparticles, on the surfaces of which blood coagulation inhibitors are immobilized, wherein the microparticles are embedded solely on the interior of the prosthesis wall and the proportion of microparticles in the prosthesis is from 1.5 to 30% by weight.

2. Vascular prosthesis according to Claim 1, **characterized in that** the blood coagulation inhibitors are immobilized on the microparticle surfaces via linkers, wherein the linkers are preferably polyalkylene glycols, more particularly polyethylene glycol (PEG).

3. Vascular prosthesis according to Claim 1 or 2, **characterized in that** the blood coagulation inhibitors are endogenous inhibitors, more particularly antithrombin III and/or hirudin.

4. Vascular prosthesis according to any of the preceding claims, **characterized in that** the microparticles are formed from polyalkyl acrylate, more particularly polymethyl methacrylate.

5. Vascular prosthesis according to any of the preceding claims, **characterized in that** the microparticles are embedded solely in an inner layer of the prosthesis wall, wherein the interior of the prosthesis wall is preferably loaded with the microparticles up to a thickness of from 0.01 to 0.2 mm.

6. Vascular prosthesis according to any of the preceding claims, **characterized in that** the interior of the prosthesis wall is formed from a layer of microparticles.

7. Vascular prosthesis according to any of the preceding claims, **characterized in that** the microparticles are from 1 to 200 µm, preferably from 5 to 60 µm, in size.

8. Vascular prosthesis according to any of the preceding claims, **characterized in that** the prosthesis is a textile vascular prosthesis, preferably a non-woven prosthesis.

9. Vascular prosthesis according to any of the preceding claims, **characterized in that** the prosthesis wall is formed by a sprayed nonwoven.

10. Vascular prosthesis according to any of the preceding claims, **characterized in that** the prosthesis wall is substantially formed from polyurethane, more particularly non-crosslinked polyurethane.

11. Vascular prosthesis according to any of the preceding claims, **characterized in that** the interior of the prosthesis wall is formed from a polymer different from the rest of the wall.

12. Method for producing the vascular prosthesis according to any of the preceding claims, **characterized in that** the microparticles are integrated into the prosthesis wall during production of the prosthesis wall and the microparticles are loaded with the blood coagulation inhibitors after completion of the prosthesis wall, wherein, firstly, a polymer dispersion containing the microparticles is applied, preferably sprayed, onto an especially rotatable mould core to form a porous layer which forms the interior of the prosthesis wall and, afterwards, the rest of the prosthesis wall is formed as a sprayed nonwoven containing no microparticles.

## Revendications

1. Prothèse vasculaire comportant une paroi poreuse à base de polymère synthétique, **caractérisée en ce que** dans la paroi sont incluses des microparticules à la surface desquelles sont immobilisés des inhibiteurs de coagulation du sang, les particules étant incluses exclusivement sur la face interne de la paroi de la prothèse et la proportion des microparticules dans la prothèse étant de 1,5 à 30 % en poids.

2. Prothèse vasculaire selon la revendication 1, **caractérisée en ce que** les inhibiteurs de coagulation du sang sont immobilisés par l'intermédiaire d'éléments de liaison sur les surfaces des microparticules, les éléments de liaison consistant de préférence en des polyalkylèneglycols, le polyéthylèneglycol (PEG) en particulier.

3. Prothèse vasculaire selon la revendication 1 ou 2, **caractérisée en ce que** les inhibiteurs de coagulation du sang consistent en des inhibiteurs endogènes, en particulier l'antithrombine III et/ou l'hirudine.

4. Prothèse vasculaire selon l'une quelconque des revendications précédentes, **caractérisée en ce que** les microparticules sont constituées de poly(acrylate d'alkyle), en particulier de poly(méthacrylate de méthyle).

5. Prothèse vasculaire selon l'une quelconque des revendications précédentes, **caractérisée en ce que** les microparticules sont incluses exclusivement dans une couche interne de la paroi de la prothèse, la face interne de la paroi de la prothèse étant chargée avec les microparticules de préférence jusqu'à une épaisseur de couche de 0,01 à 0,2 mm.

6. Prothèse vasculaire selon l'une quelconque des revendications précédentes, **caractérisée en ce que** la face interne de la paroi de la prothèse est constituée d'une couche de microparticules.

7. Prothèse vasculaire selon l'une quelconque des revendications précédentes, **caractérisée en ce que** les microparticules ont une taille de 1 à 200 µm, de préférence de 5 à 60 µm.

8. Prothèse vasculaire selon l'une quelconque des revendications précédentes, **caractérisée en ce que** la prothèse est une prothèse vasculaire textile, de préférence une prothèse non-tissée.

9. Prothèse vasculaire selon l'une quelconque des revendications précédentes, **caractérisée en ce que** la paroi de la prothèse est constituée d'un non-tissé lié par pulvérisation.

10. Prothèse vasculaire selon l'une quelconque des revendications précédentes, **caractérisée en ce que** la paroi de la prothèse est essentiellement constituée de polyuréthane, en particulier de polyuréthane non réticulé.

11. Prothèse vasculaire selon l'une quelconque des revendications précédentes, **caractérisée en ce que** la face interne de la paroi de la prothèse est constituée d'un autre monomère que la paroi restante.

12. Procédé pour la fabrication de la prothèse vasculaire selon l'une quelconque des revendications précédentes, **caractérisé en ce qu'**on intègre les microparticules dans la paroi de la prothèse lors de la fabrication de celle-ci et on charge les microparticules avec les inhibiteurs de coagulation du sang après la fabrication de la paroi de la prothèse, d'abord en appliquant de préférence par pulvérisation une dispersion de polymère contenant les microparticules sur un noyau de moule en particulier pouvant être mis en rotation, avec formation d'une couche poreuse qui constitue la face interne de la paroi de la prothèse, et ensuite en formant la paroi restante de la prothèse sous forme de non-tissé lié par pulvérisation, ne contenant pas de microparticules.
